# EUROPEAN PATENT APPLICATION

(11) **EP 4 015 011 A1**
(43) Date of publication of application: **22.06.2022**
(21) Application number: 20214589.2
(22) Date of filing: 16.12.2020
(51) Int. Cl.: A61M 1/06

(54) **BREAST PUMP SYSTEM**

(71) Applicant: Koninklijke Philips N.V., 5656 AG Eindhoven (NL)
(72) Inventor: VAN VELDHUIZEN, Gijsbert Hendrik, 5656 AE Eindhoven (NL); CLAASSEN, Coen Petrus Martinus, 5656 AE Eindhoven (NL)
(74) Representative: Philips Intellectual Property & Standards

(57) **Abstract**

A breast pump system comprises first and second expression kits. The expression kits communicate with each other. For example, this communication may be used so that first and second cyclic pressure waveforms are synchronized in order to reduce the impact of noise made by the overall system.

## Description

### FIELD OF THE INVENTION

This invention relates to breast pump systems, in particular with an expression kit for each breast, and to a breast pump for use in such a system.

### BACKGROUND OF THE INVENTION

Breast pumps are used by breast feeding women to extract milk such that the extracted milk can be fed to their babies at a later time.

Typically, the breast is placed into a funnel shaped cup and a vacuum is applied such that milk is extracted.

In a basic system, the breast pump applies a baseline vacuum. In advanced systems, in which a cyclic pressure waveform is generated, the breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

Wearable breast pumps are characterized in that they can be fully worn on the user's body, allowing the user to move around freely. Some of these are small enough to fit into a user's bra. A motor, battery and milk container are for example integrated to form a single unit.

Often, it is desirable to express both breasts at the same time. A user will then typically acquire a set of identical devices to achieve a setup which performs stimulation and expression independently for each breast.

One issue is that this results in duplication of components which may not need to be duplicated.

Another issue is that when being used, standalone wearable devices are likely to generate noise. Noise can be prevented by design by using different components, but this will increase the product cost and hence price strongly. The user will also experience a suction interaction at the breast during the expression cycle. This noise and suction feeling are dependent on the selected setting, start time of the cycle and length of the cycle.

When using two standalone devices at the same time, the noise and suction of the two devices is very likely to be not in sync resulting in a non-uniform sound, mismatch in cycle sounds and mismatch in suction feeling at both breasts. This may be disturbing for the user both in terms of the sound and the comfort.

### SUMMARY OF THE INVENTION

The invention is defined by the claims.

According to examples in accordance with an aspect of the invention, there is provided a breast pump system comprising:
a first expression kit having a first controller; and
a second expression kit having a second controller, wherein:
   the first controller is adapted to send first system data relating to control settings associated with the first expression kit or relating to sensor data received at the first expression kit to the second controller; and
   the second controller is adapted to select control settings associated with the second expression kit based on the first system data.

In this breast pump system, there are two expression kits. One can send system data (first system data) to the other for use in the control of the other expression kit. One possible purpose explained below is to allow synchronization between the two expression kits. Another possible purpose is for the first expression kit to share sensor data with the second expression kit, when sensor data received at one expression kit is equally valid at the other. This for example applies to a motion sensor or inclination sensor, or an ambient noise sensor. In this way, unnecessary duplication of components such as sensors is avoided.

The expression kit comprises at least an interface for interfacing with the breast of user, such as a breast shield. This may also optionally comprise a milk valve, optionally a membrane, and optionally a milk container. In a non-wearable design, the expression kit is typically remote from the vacuum source. In a wearable design, the expression kit may be considered to be the parts that are cleaned, such as the breast shield, and the housing with the motor unit may be considered to be a separate part. Thus, the expression kit includes at least the part that physically engages with the user. The expression kit may form an active cup or a passive cup.

The controller of each expression kit is at least able to implement the communication (i.e. transmitting of data and/or receiving of data). It may implement other functions such as control of the pressure applied to the breast (e.g. a static baseline pressure and/or a function of pressure over time).

The first controller may be a master and the second is then a slave so that communication is only needed from the first expression kit to the second. However, there is preferably bidirectional sharing of system data. In this case, the second controller is adapted to send second system data relating to control settings associated with the second expression kit or sensor data received at the second expression kit to the first controller and the first controller is adapted to select control settings associated with the first expression kit based on the second system data

The two expression systems thus then share and exchange data between them.

In a most basic implementation, the expression kit applies a base vacuum level. The communication between the controllers may then be to synchronize the base vacuum level between the two expression kits.

However, in a more advance implementation, the first expression kit has a pump and the first controller is for generating a first cyclic pressure waveform and the second expression kit has a pump and the second controller is for generating a second cyclic pressure waveform.

A first use is then for noise control. The system data (the first or second system data) is then for synchronizing the first and second cyclic pressure waveforms.

In a first approach, the synchronizing involves adapting a timing of at least one of the first and second cyclic pressure waveforms and/or adapting a shape of at least one of the first and pressure waveforms, thereby to provide synchronizing between the start times of each cycle of the first and second cyclic pressure waveforms.

In this way, each cycle of the pair is made to start at the same time. This produces a more regular noise profile, rather than a noise profile from two non-synchronized sources. The non-synchronized noises could for example result in beat frequencies and hence undesirable noise levels and noise characteristics. By aligning the start times, a regular sound pattern is created, which may be more soothing to the mother.

The two cyclic waveforms thus may originally have different cycle frequencies, and then at least one is adapted to make the cycle frequencies match. This may be achieved by adding a small delay at the end of each cycle (of the higher frequency waveform). Thus, each cycle of one of the first and second cyclic pressure waveforms may be extended to enable said synchronizing between the start times.

In a second approach, the synchronizing involves adapting at least a timing of at least one of the first and second cyclic pressure waveforms, thereby to provide an anti-phase relationship between the first and second cyclic pressure waveforms.

If the cyclic waveforms have the same cycle frequency, the adaptation may simply be a timing adaptation. Thus, the synchronizing system may then be adapted to synchronize the first and second cyclic pressure waveforms by time shifting one of the first and second cyclic pressure waveforms to enable said anti-phase relationship.

If the cyclic waveforms have different cycle frequency, the synchronizing system may further be adapted to extend each cycle of one of the first and second cyclic pressure waveforms to provide the same adapted cycle frequency (as explained above) and then enable said anti-phase relationship.

By providing anti-phase noise profiles, the loudness peaks may be reduced and a more uniform sound is created. However, the cycle frequencies are the same so that beats are again avoided.

The controllers of the first and second expression kits implement a communications system for communicating between the first and second expression kits and this communications system may be implemented with no intermediary. The communications system is preferably a wireless link.

In this case, there is no need for an overall external controller. Instead, the communication between the two kits provides the required sharing of system data, for example for synchronization. One may function as a master and the other may function as a slave. However, the controllers of the first and second expression kits may be identical so that the master slave relationship is established by communication rather than by the fixed hardware design of the expression kits. The controllers of the two expression kits may even be identical.

In this way, either of the expression kits may be used on its own, and another may be added when two kits are to be used in the synchronized way.

The controllers of the first and second expression kits may instead implement a communications system for communicating with an external controller device, wherein the control settings are implemented by a computer program which is adapted to be run on the external controller device thereby to implement the synchronization. The external controller device is for example a mobile phone. It then functions also as a user interface, providing feedback to the user and/or receiving control instructions from the user.

Each expression kit is preferably attached to a respective milk collecting vessel. The expression kit and vessel thus function as a standalone unit. The first and second expression kits are preferably wearable and they may then may fit in a feeding bra.

The invention also provides a breast pump for use as part of the breast pump system as defined above , the breast pump comprising:
an expression kit having a controller,
wherein the controller is adapted to send system data relating to control settings associated with the expression kit or sensor readings received at the expression kit to a second controller of a second expression kit for use in selecting control settings associated with the second expression kit; or
an expression kit (30) having a controller,
wherein the controller is adapted to receive system data relating to control settings associated with a second expression kit or sensor readings received at the second expression kit from a second controller of the second expression kit for use in selecting control settings associated with the first expression kit.

The invention also provides a method of controlling a breast pump system which comprises a first expression kit having a first controller and a second expression kit having a second controller, the method comprising:
the first controller sending first system data relating to control settings associated with the first expression kit or sensor readings received at the first expression kit to the second controller; and
the second controller selecting control settings associated with the second expression kit based on the first system data.

The method may comprise:
the second controller sending second system data relating to control settings associated with the second expression kit or sensor readings received at the second expression kit to the first controller; and
the first controller selecting control settings associated with the first expression kit based on the second system data.

The sharing of system data (either unidirectionally or bidirectionally) may be used for noise control.

The first controller may be for generating a first cyclic pressure waveform and the second controller is for generating a second cyclic pressure waveform, wherein the method comprises synchronizing the first and second cyclic pressure waveforms by:
adapting a timing of at least one of the first and second cyclic pressure waveforms and/or adapting a shape of at least one of the first and second cyclic pressure waveforms, thereby to provide synchronizing between the start times of each cycle of the first and second cyclic pressure waveforms; or
adapting at least a timing of at least one of the first and second cyclic pressure waveforms, thereby to provide an anti-phase relationship between the first and second cyclic pressure waveforms.

The method may comprise synchronizing the first and second cyclic pressure waveforms by extending each cycle of one of the first and second cyclic pressure waveforms to enable said synchronizing between the start times.

The method may instead comprise synchronizing the first and second cyclic pressure waveforms by:
time shifting one of the first and second cyclic pressure waveforms to enable said anti-phase relationship; and optionally
extending each cycle of one of the first and second cyclic pressure waveforms to enable said anti-phase relationship.

Implementing the synchronization may be by:
communication between the controllers of the first and second expression kits with no intermediary; or
communication with an external controller device.

The invention also provides a computer program comprising computer program code means which is adapted, when said program is run on a processor, to implement the method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a mother wearing two wearable breast pumps;
Figure 3 shows a first example of breast pump system in accordance with the invention; and
Figure 4 shows a second example of breast pump system in accordance with the invention; and
Figure 5 is used to explain the noise control implemented by the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system which has first and second expression kits. The expression kits communicate with each other. For example, this communication may be used so that first and second cyclic pressure waveforms are synchronized in order to reduce the impact of noise made by the overall system.

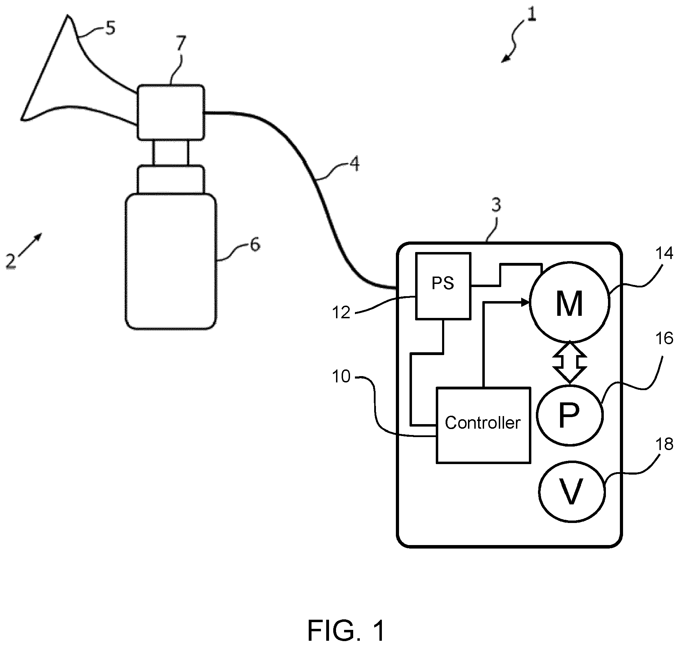

Figure 1 shows a known breast pump system 1, comprising an expression unit 2 and a pump arrangement 3 which are connected via a tube 4. The pump arrangement includes various components in addition to the pump, so may be considered to be a general operating unit.

The expression unit 2 is formed with a main body 7, a funnel 5 for receiving a breast of a user and a receptacle 6 for collecting the expressed milk. The funnel 5 and the receptacle 6 are connected to the main body 7. The main body 7 comprises a vacuum chamber. A flexible membrane or diaphragm is located in the vacuum chamber. The membrane prevents expressed milk from flowing into the tube 4 leading to the pump arrangement unit 3.

The pump arrangement 3 may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16. The controller controls 10 the operation of the power source 12, motor 14 and vacuum pump 16. The pump arrangement 3 further comprises a solenoid valve 18.

In use, the vacuum pump applies a vacuum to the membrane located in the main body 7 so that it deforms. The membrane deforms to create a vacuum in the funnel 5 which in turns applies a vacuum to the breast which enables milk to be expressed. Although the breast pump system 1 is described as comprising a membrane such that the vacuum is applied indirectly to the breast, it should be understood that in an alternative embodiment, the vacuum is applied directly to the breast of a user. In this case, the breast pump system does not comprise a membrane and the vacuum created by the vacuum pump is applied directly to the breast.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects to the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection vessel. This enables breast pumping to be performed more discretely.

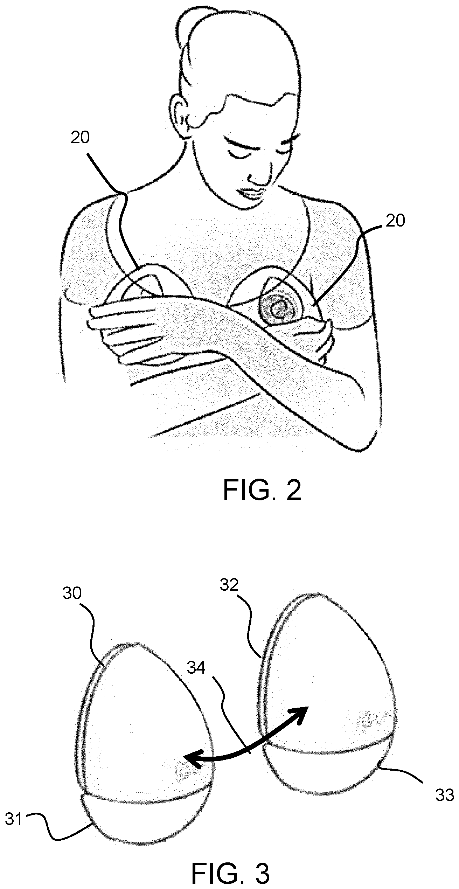

Figure 2 shows a mother wearing two wearable breast pumps 20.

In such a design, the funnel 5 of Figure 1 is implemented as breast shield, and this breast shield is received within an outer enclosure of the wearable breast pump.

Figure 3 shows a first example of breast pump system, comprising a first expression kit 30 and a second expression kit 32. Each expression kit is a wearable device, and it is attached to a respective milk collection vessel 31,33. Each expression kit is fitted over a breast shield.

The expression kits may each comprise a milk sensor for sensing milk flow and for measuring flow volume. Each expression kit has a pump and a controller for generating a cyclic pressure waveform. Thus, the first expression kit has a first controller for generating a first cyclic pressure waveform and the second expression kit has a second controller for generating a second cyclic pressure waveform.

Different pressure waveforms may for example be selected by a user, for example different intensity levels, depending on the level of comfort of the mother to those different intensity levels.

The first controller of the first expression kit sends first system data relating to control settings of the first expression kit or sensor readings received at first expression kit to the second controller of the second expression kit. The second controller then uses this first system data to select control settings associated with the second expression kit. Thus, the second expression kit is not controlled as an independent unit, but its control depends on that of the first expression kit. However, instead of a global system controller sending control commands to both expression kits, one sends its own system data to the other, thus implementing a control loop between the two expression kits.

The second controller of the second expression kit may also send second system data relating to control settings of the second expression kit or sensor readings received at the second expression kit to the first controller of the first expression kit. The first controller then uses this first system data to select control settings associated with the first expression kit.

Thus, in this case, system data is shared between the two expression kits and one or both can adapt their local settings in dependence on the system date of the other.

The system data may be the settings selected by the user, such as an intensity level or a mode of operation, or it may be state information such as timing information or pressure level information. The system data may include sensor data such as pressure sensor data, motion or orientation sensor data, microphone sensor data etc.

In one set of examples, in which the breast pumps each implement a cyclic pressure waveform, the communications channel implements a synchronizing system for synchronizing the first and second cyclic pressure waveforms.

In Figure 3, the communications channel and hence synchronizing system is represented as a communication link 34 between the expression kits. Using this link 34, intensity settings and timing information may be shared between the two kits.

The two expression kits may be different, and the communication link is used to enable these differences to be tolerated.

The differences may arise because the two expression kits are of different design. Thus, even if both are set to a same intensity setting, they may deliver different cyclic pressure profiles. Even if the two expression kits are of the same design, process spread may mean that the cyclic pressure profiles are not the same. One controller may have a different clock speed to the other, so that even if the same pressure profile is instructed by each controller, timing spread may result.

When an expression kit is set to a different intensity setting, the pressure profile typically also changes, with different timing, because a different time is needed for the different pressure levels to be reached during application of the pressure profile. Thus, if two expression kits are set to different intensity levels, the timing of the pressure profiles is likely to differ.

By sharing system data, such as intensity setting information between the expression kits (or at least from one to the other) synchronization can take place. For example, one expression kit can periodically send a notification identifying the start of its expression cycle to the other expression kit. This timing information may then be part of the transferred system data.

As another example, sensor data from one expression kit may be shared with the other so that duplication of sensors is avoided. This sensor data is then part of the system data. This may be sensor data relating to the milk ejection reflex (which arises in both breasts at the same time) or motion or orientation sensing in respect of the mother (which only needs to be sensed at one location) or container level sensing so that one expression kit can know about the state of the expression of the other expression kit.

At least one expression kit generates an output for delivery of data (directly or via an external intermediary) to the other. The output conveys the system data as explained above

At least one expression kit (the other one) has an input for receiving the delivered data (directly or via an external intermediary) to the other. The received data is again the system data discussed above.

Preferably, the communication link is bidirectional so each expression kit can send system data to, and receive system data from, the other.

In this way, the two expression kits are not independently controlled, either by respective local controllers or by a shared system controller. Instead, the overall system control is based on communication between two local controllers.

The communication link 34 is for example a Bluetooth connection directly between the kits, with no communication to an external controller. One or both kits may output status information to a remote device, but in this example the control functions are implemented by the devices themselves. A different communication technology may of course be used such IR, NFC, RF or other.

One kit may function as a master and the other may function as a slave. However, the first and second expression kits may be identical so that the master-slave relationship is established by communication rather than by the fixed hardware design of the expression kits.

In this way, either of the expression kits may be used on its own, and another may be added when two kits are to be used in the synchronized way. There may instead be separate master and master and slave device designs.

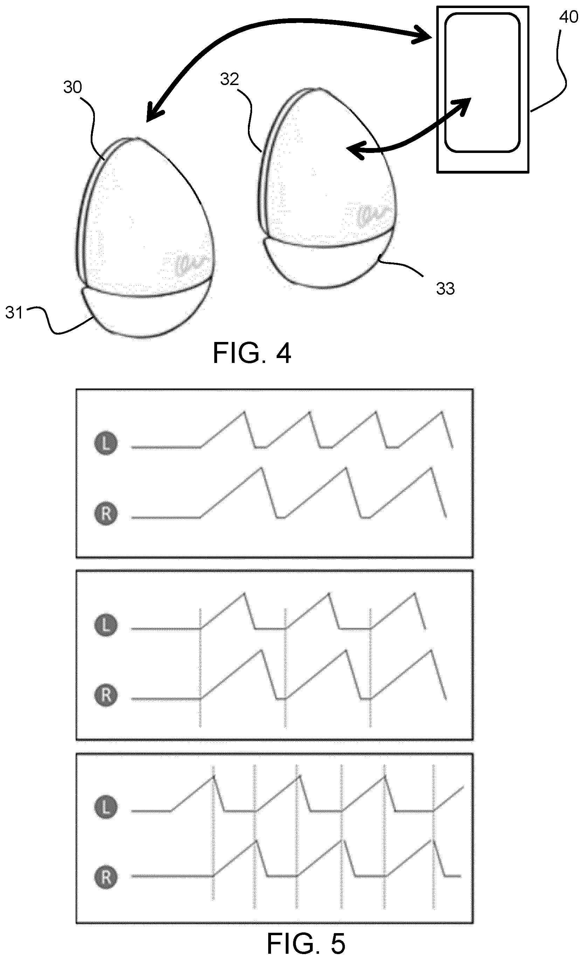

Figure 4 shows a second example of breast pump system, again comprising a first wearable expression kit 30 and a second wearable expression kit 32, each with a respective milk collection vessel 31,33.

In this example, the first and second expression kits each have a communications system for communicating with an external controller device 40. The control system then includes a computer program (app) which is run on the external controller device such as a mobile phone. The mobile phone functions also as a user interface, providing feedback to the user and/or receiving control instructions from the user.

As mentioned above, in some examples, the synchronization is used to control the output noise in a desired way. Two possible synchronization functions are explained with reference to Figure 5.

The top pane shows in simplified form two noise profiles, in which the rising and falling ramps coincide with phases of the pressure cycle.

The two noise profiles have different cycle frequency and different peak amplitude, because the pressure cycles, e.g. selected by user, have different pressure levels and cyclic frequency. These differences may relate to different modes (e.g. expression mode and stimulation mode) or different settings within a mode e.g., with the expression mode. These settings may be selected by the user for optimal comfort or for controlling the time of the expression process and they may be different for the two breasts.

Different intensity settings for example result in different periods for the cyclic waveforms, as shown in the top pane of Figure 5.

The middle pane shows adaptation of the upper noise profile (by adapting the corresponding pressure profile) according to a first approach. In this first approach, the transfer of system data implements a synchronization, which involves adapting a timing of at least one of the first and second pressure waveforms and adapting a shape of at least one of the first and second cyclic pressure waveforms (the top one in the figure in this example) so that the start times of each cycle of the first and second cyclic pressure waveforms are synchronized, as shown.

The timing alignment may be reset periodically for example to take account of possible variations in clock speed of the two controllers.

In this way, each cycle of the pair is made to start at the same time. This produces a more regular noise profile, rather than a noise profile from two out of synchronization sources. This avoids beat frequencies and hence undesirable noise levels and noise characteristics. By aligning the start time, a regular sound pattern is created, which may be more soothing to the mother.

The breast feeding sensation may also be improved by having a regular repeating pattern which is in synchronism between the two breasts.

The timing synchronization according to this example thus goes beyond simply aligning the timing of two identical expression kits. Instead, the two cyclic waveforms originally may have different cycle frequencies, so that shape adaptation is needed to make the cycle frequencies match. This is achieved in Figure 5 by adding a small delay at the end of each cycle of the one with higher cycle frequency (the top one). This may for example be the one with lower intensity setting, since shorter times are needed to reach the intended pressure levels. Thus, each cycle of one of the first and second cyclic pressure waveforms is extended to enable said synchronizing between the start times.

The bottom pane shows a second approach in which (simply by way of example) the original pressure waveforms have the same cyclic frequency. The synchronizing involves adapting a timing of at least one of the first and second cyclic pressure waveforms (e.g. the lower one) to provide an anti-phase relationship between the first and second cyclic pressure waveforms and hence between the noise profiles.

When the cyclic waveforms have the same cycle frequency, the adaptation may simply be a timing adaptation of time shifting one of the first and second cyclic pressure waveforms to enable said anti-phase relationship.

If the cyclic waveforms have different cycle frequency, the synchronizing system may also extend each cycle of one of the first and second cyclic pressure waveforms as explained above. to provide the same adapted cycle frequency and hence enable said anti-phase relationship.

By providing anti-phase noise profiles, the loudness peaks may be reduced and a more uniform sound is created. However, the cycle frequencies are the same so that beats are again avoided.

As explained above, different modes and settings have different cyclic frequency and different maximum under-pressure (i.e. vacuum) levels.

The expression mode for example cycles between a baseline vacuum and a maximum vacuum (i.e. a maximum under-pressure) "Max Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline Vac = atmospheric pressure
Max Vac = 300 mbar (30 kPa) below atmospheric pressure
TTV = 0.85 s
DI = 0.3 s
TTA = 0.05 s
DO = 0.35 s

This gives a total cycle time T_cycle of 1.55s.

These values are simply by way of example. The maximum under-pressure may be higher, for example 350 mbar (35 kPa) and the durations may differ. The cycle time is generally of the order of 1 to 2 seconds.

The stimulation mode for example has a faster frequency hence with a shorter cycle time of around 0.5 seconds, and a lower vacuum, for example a maximum under-pressure level of 150 mbar (15 kPa).

In an expression kit with both stimulation mode and expression modes, the stimulation mode for example takes placing during an initial period of the breast pumping session (e.g. 50 seconds) and the expression mode takes place during the remainder of the breast pumping session.

There may also be different settings of expression mode, again with different characteristics.

A controller may also implement a positioning mode. This may comprise intermittent application of a small under-pressure to allow the user to position the breast shield while observing the correct alignment. A controller may also implement a massage mode.

The noise control functionality explained above may be applied to all mode.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system comprising:
a first expression kit (30) having a first controller; and
a second expression kit (32) having a second controller, wherein:
the first controller is adapted to send first system data relating to control settings associated with the first expression kit or relating to sensor data received at the first expression kit to the second controller; and
the second controller is adapted to select control settings associated with the second expression kit based on the first system data.

2. The system of claim 1, wherein:
the second controller is adapted to send second system data relating to control settings associated with the second expression kit or sensor data received at the second expression kit to the first controller; and
the first controller is adapted to select control settings associated with the first expression kit based on the second system data

3. The system of claim 1 or 2, wherein:
a first expression kit (30) has a pump and the first controller is for generating a first cyclic pressure waveform; and
the second expression kit (32) has a pump and the second controller is for generating a second cyclic pressure waveform.

4. The system of claim 3, wherein the system is adapted to synchronize the first and second cyclic pressure waveforms is by adapting a timing of at least one of the first and second cyclic pressure waveforms and/or adapting a shape of at least one of the first and second cyclic pressure waveforms, thereby to provide synchronizing between the start times of each cycle of the first and second cyclic pressure waveforms.

5. The system of claim 4, wherein the synchronizing of the first and second cyclic pressure waveforms is by:
extending each cycle of one of the first and second cyclic pressure waveforms to enable said synchronizing between the start times; or
adapting at least a timing of at least one of the first and second cyclic pressure waveforms, thereby to provide an anti-phase relationship between the first and second cyclic pressure waveforms.

6. The system of claim 5, wherein the synchronizing of the first and second cyclic pressure waveforms is by time shifting one of the first and second cyclic pressure waveforms to enable said anti-phase relationship and optionally further by extending each cycle of one of the first and second cyclic pressure waveforms to enable said anti-phase relationship.

7. The system of any one of claims 1 to 6, wherein the first and second controllers are configured to communicate between the first and second expression kits with no intermediary.

8. The system of claim 7, wherein:
the first and second controllers have identical communication functionality; or
the first expression kit is a controller expression kit and the second expression kit is a slave expression kit.

9. The system of any one of claims 1 to 8, wherein the first and second expression kits each have a communications system for communicating with an external controller device, wherein the control settings are implemented by a computer program which is adapted to be run on the external controller device.

10. The system of any one of claims 1 to 9, wherein each expression kit is attached to a respective milk collecting vessel.

11. A breast pump for use as part of the breast pump system of any one of claims 1 to 10, the breast pump comprising:
an expression kit (30) having a controller,
wherein the controller is adapted to send system data relating to control settings associated with the expression kit or sensor readings received at the expression kit to a second controller of a second expression kit for use in selecting control settings associated with the second expression kit; or
an expression kit (30) having a controller,
wherein the controller is adapted to receive system data relating to control settings associated with a second expression kit or sensor readings received at the second expression kit from a second controller of the second expression kit for use in selecting control settings associated with the first expression kit.

12. A method of controlling a breast pump system which comprises a first expression kit having a first controller and a second expression kit having a second controller, the method comprising:
the first controller sending first system data relating to control settings or associated with the first expression kit or sensor readings received at the first expression kit to the second controller; and
the second controller selecting control settings associated with the second expression kit based on the first system data.

13. The method of claim 12, wherein the first controller is for generating a first cyclic pressure waveform and the second first controller is for generating a second cyclic pressure waveform, wherein the method comprises synchronizing the first and second cyclic pressure waveforms by:
adapting a timing of at least one of the first and second cyclic pressure waveforms and/or adapting a shape of at least one of the first and second cyclic pressure waveforms, thereby to provide synchronizing between the start times of each cycle of the first and second cyclic pressure waveforms; or
adapting at least a timing of at least one of the first and second cyclic pressure waveforms, thereby to provide an anti-phase relationship between the first and second cyclic pressure waveforms.

14. The method of claim 13, comprising synchronizing the first and second cyclic pressure waveforms by:
extending each cycle of one of the first and second cyclic pressure waveforms to enable said synchronizing between the start times; or
time shifting one of the first and second cyclic pressure waveforms to enable said anti-phase relationship and optionally extending each cycle of one of the first and second cyclic pressure waveforms to enable said anti-phase relationship.

15. A computer program comprising computer program code means which is adapted, when said program is run on a processor, to implement the method of any one of claims 12 to 14.
